# EUROPEAN PATENT APPLICATION

(11) **EP 4 400 156 A1**
(43) Date of publication of application: **17.07.2024**
(21) Application number: 23219718.6
(22) Date of filing: 22.12.2023
(51) Int. Cl.: A61N 1/32, A61N 1/04

(54) **TREATMENT APPARATUS FOR DENATURING TISSUE USING RF ENERGY AND CONTROL METHOD FOR THAT**

(30) Priority: 12.01.2023 KR 20230004910; 26.10.2023 KR 20230144571
(71) Applicant: Lutronic Corporation, Goyang-si, Gyeonggi-do 10534 (KR)
(72) Inventor: KO, Kwangchon, 10394 Goyang-si, Gyeonggi-do (KR)
(74) Representative: Michalski Hüttermann & Partner Patentanwälte mbB

(57) **Abstract**

The present disclosure relates to a skin treatment device for denaturing tissue using multi-frequency RF energy, which performs skin treatment using RF energy of various frequencies for delivering energy to different depths within the tissue, a control method thereof.

## Description

### CROSS-REFERENCE TO RELATED APPLICATIONS

Priority to Korean patent application number 10-2023-0004910 filed on January 12, 2023 and Korean patent application number 10-2023-0144571 filed on October 26, 2023 the entire disclosure of which is incorporated by reference herein, is claimed

### TECHNICAL FIELD

The present disclosure relates to a treatment apparatus for denaturing tissue using RF(Radio-Frequency) energy and control method for that.

### BACKGROUND

Devices for delivering RF energy to tissue for therapeutic purposes have been developed in various manners. In particular, devices that cause appropriate degeneration of the skin using RF energy and exert a skin treatment effect through tissue regeneration have recently been developed.

Korea Patent No. 0706155 is disclosed in relation to a conventional treatment device using RF energy. This conventional device has a problem of low treatment efficiency because the skin is treated using RF energy at a fixed frequency.

### SUMMARY

To solve the aforementioned problem, the present disclosure provides a treatment apparatus for denaturing tissue using RF energy which can adjust the frequency of RF energy in response to the depth and temperature of tissue, a control method thereof.

In an aspect, there is provided a treatment device using RF energy which can adjust RF energy to multiple frequencies and delivering the RF energy during one-time treatment.

Multiple frequencies of RF energy may include at least three frequencies for different heating depths within the tissue.

Additionally, RF energy may be delivered to the tissue according to a pulse train, and the frequency of selecting a frequency of the RF energy may be controlled through the pulse train on the basis of at least one of an elapsed time of treatment, a tissue temperature, or a treatment mode.

Additionally, according to the present disclosure, a method of controlling a skin treatment device for denaturing tissue using multi-frequency RF energy is provided.

Additionally, according to the present disclosure, there is provided a method of denaturing tissue by configuring a pulse train with frequencies for different heating depths in skin tissue and delivering RF energy to the tissue according to the pulse train. The method of denaturing tissue can improve treatment efficiency by adjusting the frequency of delivering RF energy to the epidermis layer, dermis layer, and fat layer on the basis of least one of an elapsed time of treatment, the temperature of tissue or skin surface, and a treatment mode.

According to the skin treatment device for denaturing tissue using multi-frequency RF energy, the control method thereof, and the method of denaturing tissue using the same according to the present disclosure, it is possible to induces regeneration for producing new collagen fibers by denaturing the epidermis and dermis with RF energy and to remodeling the skin contour by killing fat cells with the RF energy.

### BRIEF DESCRIPTION OF THE DRAWINGS

FIG. 1 is a block diagram showing a configuration of a skin treatment device for denaturing tissue using multi-frequency RF energy according to a first embodiment of the present disclosure.
FIG. 2 is a diagram illustrating areas in skin tissue which are heated according to frequencies of RF energy selected in the first embodiment.
FIG. 3 is a diagram illustrating an example of a pulse train of RF energy in the first embodiment.
FIG. 4 is a graph showing a heat phase and a hold phase according to the temperature of the tissue when RF energy is delivered in the first embodiment.
FIG. 5 is a diagram showing a pulse train of RF energy, a tissue temperature, and operation time of a cooling unit in the first embodiment.
FIG. 6 is a diagram showing a heat phase pulse train in the first embodiment.
FIG. 7 is a diagram showing a hold phase pulse train in the first embodiment.
FIGs. 8 and 9 are diagrams showing pulse trains with adjusted heat phase pulse widths in the first embodiment.
FIG. 10 is a flowchart of a method of controlling a skin treatment device for denaturing tissue using multi-frequency RF energy according to a second embodiment of the present disclosure.
FIG. 11 is a detailed flowchart of the second embodiment of the present disclosure.
FIG. 12 is a flowchart of a treatment method for denaturing tissue using multi frequency RF energy, which is a third embodiment of the present disclosure.
FIG. 13 is a detailed flowchart of the third embodiment.

### DETAILED DESCRIPTION

Hereinafter, a skin treatment device for denaturing tissue using multi-frequency RF energy, a control method thereof according to embodiments of the present disclosure will be described in detail with reference to the attached drawings. In the description of the following embodiments, the name of each component may be referred to as different names in the art. However, if there is functional similarity and identity between components, they can be regarded as equivalent components even if modified embodiments are adopted. Additionally, a symbol is attached to each component for convenience of description. However, content shown in the drawings in which such symbols are written does not limit each component to the scope within the drawings. Likewise, even if an embodiment in which a configuration in a drawing is partially modified is adopted, it can be regarded as an equivalent configuration if there is functional similarity and identity. Further, if a component is recognized as a component that should be included in light of the general level of technicians in the relevant technical field, the description thereof will be omitted.

The present disclosure will be described on the premise that treatment means heating the skin tissue to improve wrinkles, tone and textural changes, scars and acne scarring, sagging mucosa, overall rejuvenation, hyperhidrosis, laxity, lifting, tightening, fat reduction, etc., and treatment means heating and killing fat cells to prevent additional fat accumulation.

Furthermore, in this specification, the term 'tissue' can refer to facial skin tissue. Additionally, 'tissue' may encompass skin tissue from various parts of the human body such as the abdomen and thighs. According to the embodiments disclosed herein, when treating facial skin tissue, positive effects such as improvement in wrinkles as mentioned earlier, and enhancement of facial contour through the apoptosis of fat cells can be achieved. Improvement in facial contour implies a change in the shape or appearance of the face, contributing to positive outcomes for the patient, such as a favorable impression or a more youthful appearance.

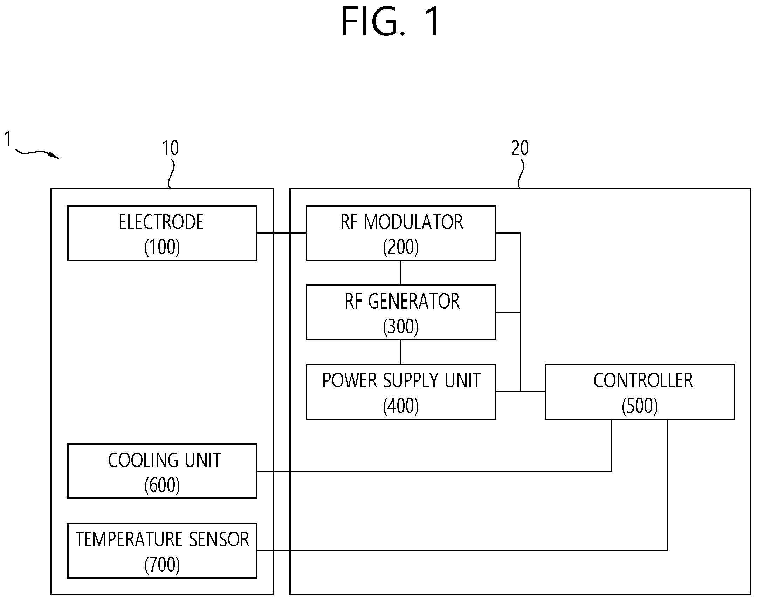

FIG. 1 is a block diagram showing a configuration of a skin treatment device for denaturing tissue using multi-frequency RF energy according to a first embodiment of the present disclosure.

Referring to FIG. 1, the skin treatment device 1 for denaturing tissue using multi-frequency RF energy according to the first embodiment of the present disclosure includes a main body 2 and a handpiece 10. The main body 2 includes elements for generating and controlling RF energy, and the handpiece 10 is configured to deliver RF energy to tissue. The main body 20 may include a power supply unit 400, an RF generator 300, an RF modulator 200, and a controller 500. The handpiece 10 may include an electrode 100, a temperature sensor 600, and a cooling unit 700. Although not shown, the handpiece 10 and the main body 20 may be connected by a cable to deliver and receive RF energy and various signals for control. Additionally, the handpiece 10 and/or the main body 20 may further include a display (not shown) for displaying various types of information related to operation and treatment and receiving commands from a user.

The RF generator 300 may be configured to receive power from the power supply unit 400 and to generate RF energy. The RF modulator 200 is configured to control at least one of the frequency, power, voltage, or electric power of the RF energy generated by the RF generator 300. Meanwhile, a well-known circuit configuration may be applied to the power supply unit 400, the RF generator 300, and the RF modulator 200.

The electrode 100 is electrically connected to the RF modulator 200 and is configured to receive RF energy. The electrode 100 is configured to be exposed at the end of the handpiece and can be configured to deliver RF energy to tissue at the time of coming into contact with the tissue. A plurality of electrodes 100 may be provided and arranged in a predetermined pattern. At least a portion of the electrode 100 may be configured as a flat surface to non-invasively deliver RF energy to the tissue. The electrode 100 may serve as a bipolar electrode or a monopolar electrode.

The cooling unit 600 may be configured to prevent excessive damage to the tissue when RF energy is delivered to the tissue to heat the tissue. The cooling unit 600 may cool the rear of the electrode in contact with the tissue or may directly cool the skin tissue. The cooling unit 600 may spray coolant to cool the electrode and/or the tissue through the heat of vaporization of the coolant. Additionally, the cooling unit 600 may selectively bring a heat sink into contact with the electrode and/or tissue to cool the electrode and/or the tissue in a conductive manner. Further, the cooling unit 600 may be configured as an air cooling type to force air to flow inside the handpiece. However, the configurations of the cooling unit 600 described above are merely examples and may be modified and applied as various configurations in which the cooling power is actively adjusted according to the configuration of the controller 500.

The temperature sensor 700 is configured to measure temperature at a plurality of points while RF energy is delivered to tissue. The temperature sensor 700 may include a plurality of temperature sensors. The plurality of temperature sensors may be configured to measure temperature at a plurality of points in a treatment area to which RF energy is delivered.

The controller 500 controls the RF modulator 200 to adjust the frequency or pulse width of RF energy. The controller 500 configures a pulse train and delivers multi-frequency RF energy through a sequence. Additionally, the controller 500 may control the RF generator 300 to block RF energy upon determining that the RF energy needs to be blocked on the basis of values received from the plurality of temperature sensors 700. Furthermore, the controller may control the operation of the cooling unit 600 according to a value received from the temperature sensor 700 or the frequency of RF energy currently applied to the tissue.

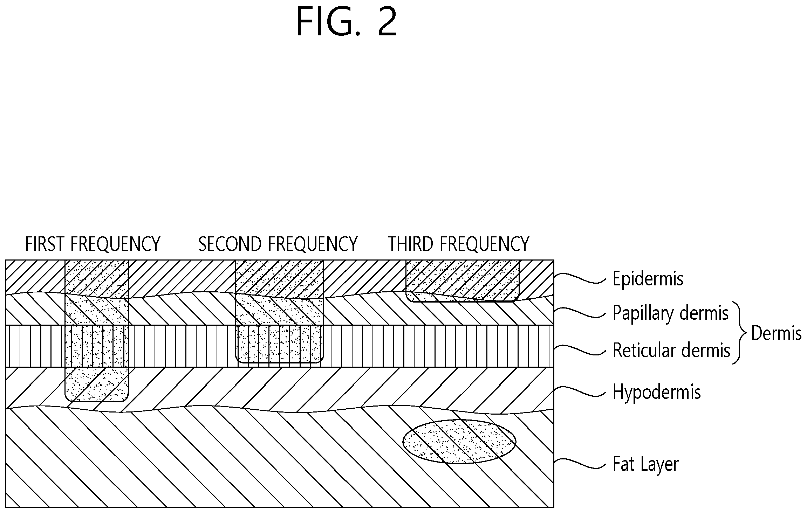

FIG. 2 is a diagram illustrating areas in the skin tissue which are heated according to frequencies of RF energy selected in the first embodiment. Here, frequency magnitudes are in the relationship of first frequency < second frequency < third frequency.

Referring to FIG. 2, when RF energy is delivered through the skin, a heating depth may vary depending on the frequency of the RF energy. When the RF electrode is configured as a monopolar electrode, RF energy is delivered from the electrode of the handpiece to a return electrode (not shown) separately attached to the human body. It has been revealed that a heated area within skin tissue varies depending on the frequency of RF energy.

Skin tissue can be divided into epidermis, dermis (papillary dermis and reticular dermis), hypodermis, and fat layer from the surface. Layers constituting the skin tissue may have different conductivities and dielectric constants for RF frequencies. By adjusting the frequency of RF energy using such electrical characteristics of the tissues, it is possible to heat the skin to a desired heating depth.

In the present disclosure, a heated area within the skin tissue can be controlled by adjusting the frequency of RF energy such that the skin treatment device can achieve optimal treatment effect.

As the frequency of RF energy increases within a certain range, the tissue is heated to a shallower depth. The controller can control a heated area within the tissue by adjusting the frequency of RF energy to the first frequency, second frequency, and third frequency. The first frequency may be selected as a frequency capable of heating the skin tissue from the epidermis to the dermis and hypodermis. The second frequency may be selected as a frequency capable of heating the skin tissue from the epidermis to the dermis. The third frequency may be selected as a frequency capable of selectively heating the deep fat layer. Here, the RF energy at the third frequency can heat the superficial portion and deep fat cells of the skin tissue. As described above, it is possible to selectively heat the deep fat layer by applying RF energy having a specific frequency according to the electrical characteristics of each layer of the skin tissue.

The first frequency may be in the range of 1 to 3 MHz, the second frequency may be in the range of 3 to 10 MHz, and the third frequency may be in the range of 10 to 30 MHz. As a more specific example, the first frequency may be 2 MHz, the second frequency may be 6 MHz, and the third frequency may be 13 MHz. As an example, the first frequency may be 2.26 MHz, the second frequency may be 6.75 MHz, and the third frequency may be 13.56 MHz.

However, the present disclosure is not limited to the frequencies described above, and in the present disclosure, the first frequency, second frequency, and third frequency can be defined as frequency regions by which a heating depth within the tissue varies, and the third frequency can be selected as a frequency range that can heat fat cells better than other layers constituting the skin.

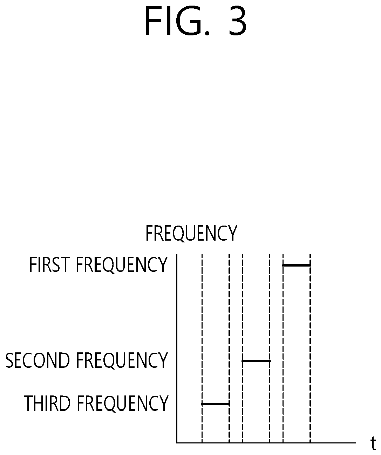

FIG. 3 is a diagram illustrating an example of a pulse train of RF energy in the first embodiment.

Referring to FIG. 3, in the first embodiment, the pulse train of RF energy may be composed of, for example, a first frequency of 1 to 3 MHz, a second frequency of 3 to 10 MHz, and a third frequency of 10 to 30 MHz.

Meanwhile, when RF energy is delivered within the skin tissue, the difference in energy delivery efficiency depending on temperature may change rapidly. This is caused by changes in impedance within the tissue depending on temperature differences. Therefore, at the time of delivering RF energy to treat deep tissue, the RF energy delivery efficiency can be improved if preheating is performed in the direction in which the RF energy is delivered.

Meanwhile, as an example of a frequency constituting a pulse train in the present disclosure, the first frequency for initially heating the epidermis, dermis, and hypodermis layers is set to 2.26 MHz. Thereafter, a predetermined rest period is provided in the pulse train, and then 6.75 MHz is selected as the second frequency for heating the epidermal layer and dermis. If the RF energy of the second frequency is delivered in a state in which the epidermis, dermis, and hypodermis layers have been heated by the RF energy of the first frequency, the efficiency of RF energy delivery to the dermis layer can be maximized. Thereafter, a predetermined rest period is provided in the pulse train, and then the third frequency for heating the fat layer may be set to 13.56 MHz. Here, since the RF energy at the first frequency and the RF energy at the second frequency have already been delivered and thus the epidermal layer and dermal layer have been heated, the electrical characteristics of each layer of the tissue change and the heat distribution pattern within the tissue changes. Finally, when the RF energy at the third frequency for heating the fat layer is applied to the skin tissue, the RF energy delivery efficiency is increased, and selective heating of the fat layer is efficiently achieved. Further, from a thermal perspective, the fat layer is heated in a state in which the hypodermis has been heated in advance, and thus heat loss from the heated fat layer to the surface can be minimized.

However, the aforementioned predetermined rest periods may be omitted. Additionally, in a case where a plurality of electrodes is configured, RF energy of different frequencies may be delivered to the electrodes. In this case, RF energy of at least two of the first frequency, second frequency, or third frequency may be overlapped and delivered to the tissue.

As described above, the present disclosure uses multi-frequency RF energy to maximize the RF energy delivery efficiency depending on heating depth. In addition, tissue adjacent to fat cells is heated according to the pulse pattern of the basic pulse train, and the frequencies of pulses of RF energy are adjusted such that the deep tissue can be heated.

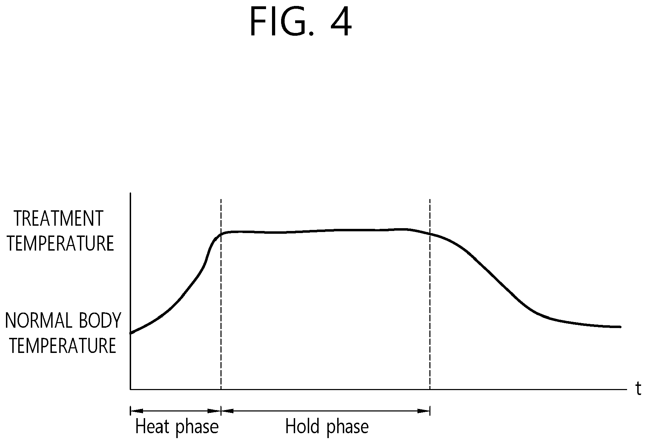

FIG. 4 is a graph showing a heat phase and a hold phase according to the temperature of tissue when RF energy is delivered in the first embodiment.

Referring to FIG. 4, when delivering RF energy to kill fat cells, a treatment interval can be distinguished according to the temperature of the fat layer. The treatment interval may be divided into a heat phase, which is an interval in which RF energy is delivered to the fat layer to heat the same, and a hold phase, which is an interval in which the fat layer is maintained at a treatment temperature. The controller may configure different pulse trains for the heat phase and the hold phase. As an example, the frequency of the first frequency may be adjusted differently or the frequency of the third frequency may be configured differently in the respective phases. As another example, pulse trains may be configured with different ratios of the first frequency: second frequency: third frequency in the heat phase and the hold phase. As an example, a pulse train may be configured with a ratio of the first frequency: second frequency: third frequency of 2: 3: 5 in the heat phase, and a pulse train may be configured with a ratio of 1: 3: 6 in the hold phase.

Additionally, at least one of pulse widths constituting a pulse train may be adjusted. That is, the pulse width of the first frequency can be increased in the heat phase, and the pulse width of the third frequency can be increased in the hold phase.

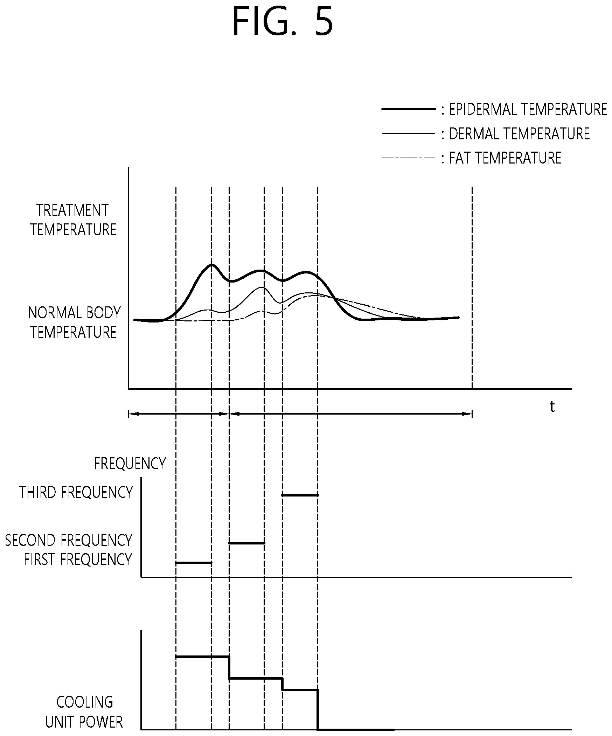

FIG. 5 is a diagram showing a pulse train of RF energy, a tissue temperature, and operation time of the cooling unit in the first embodiment.

For skin remodeling, it is necessary to heat the dermis layer to a temperature at which the dermis layer can be denatured, for example, 70 °C to 80 °C, to denature the dermis layer into a coagulation state.

Meanwhile, change in the curvature of the skin surface acts as a major factor of change in the face shape. Curvature of the skin surface can be corrected by changing the thickness of the fat layer present in the deep area in the tissue. The death of fat cells prevents additional accumulation of fat and ultimately has the effect of thinning the fat layer. It is known that heating to 44 °C to 46 °C is necessary to kill fat cells.

In the case of treating the dermis and fat of the skin simultaneously, it is necessary to denature the dermis layer and kill fat cells present in the fat layer simultaneously for skin remodeling.

If a pulse train is determined in the order of 2.26 MHz, 6.75 MHz, and 13.56 MHz, the epidermis, dermis, and hypodermis are heated first, and finally the fat layer can be heated to a treatment temperature. Here, since the temperature of the epidermal layer may continuously increase, the epidermal layer may be cooled to prevent excessive damage. The controller can adjust the power of the cooling unit on the basis of the frequencies constituting the pulse train. The controller can increase the power of the cooling unit when RF energy is delivered at a frequency of 2.26 MHz, used to heat the epidermis to the hypodermis. On the other hand, the controller can decrease the power of the cooling unit when RF energy is delivered at a frequency of 13.56 MHz at which the RF energy is concentrated on the deep area.

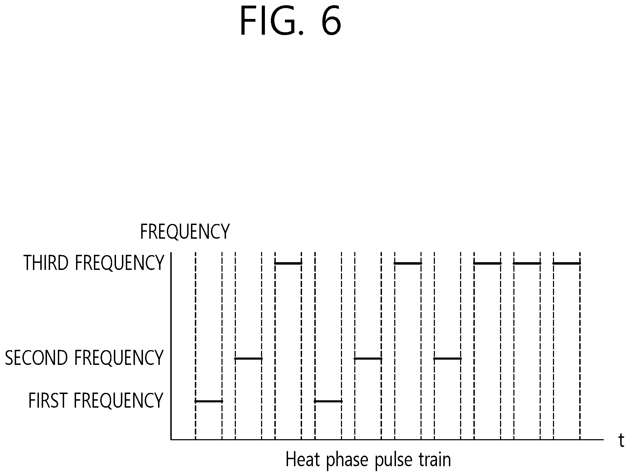

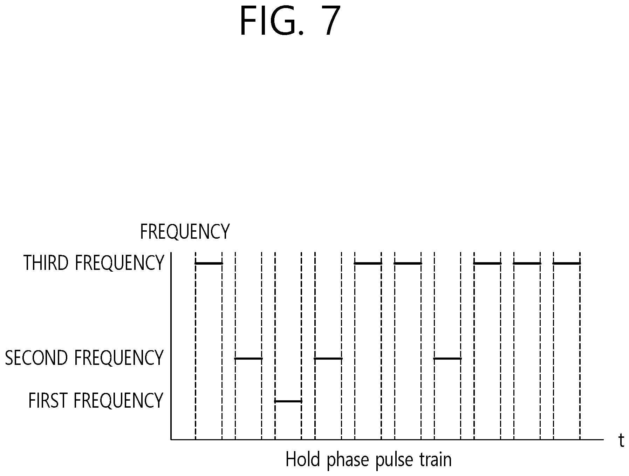

FIG. 6 is a diagram showing a heat phase pulse train in the first embodiment, and FIG. 7 is a diagram showing a hold phase pulse train in the first embodiment.

In the present disclosure, the controller may configure different pulse trains for the heat phase and the hold phase.

As an example, referring to FIG. 6, the controller may set the ratio of the first frequency: second frequency: third frequency included in the pulse train to 2:3:5 in order to rapidly heat the tissue to a treatment temperature in the heat phase. Here, when applying RF energy to the tissue for the first time, it is preferable to configure the pulse train in the order of the first frequency for heating the epidermis, dermis, and hypodermis, and the second frequency for heating the epidermis and dermis. The process of delivering RF energy in the order of the first frequency, second frequency, and third frequency may be repeated a predetermined number of times within the heat phase.

As another example, referring to FIG. 7, the controller may determine the ratio of the first frequency: second frequency: third frequency included in the pulse train as 1:3:6 in the hold phase to prevent damage while maintaining the tissue at the treatment temperature. That is, the frequency of the third frequency for delivering RF energy to the fat layer with the highest specific heat after the temperature of the tissue is raised to the treatment temperature can be set to the highest. The frequency of the third frequency in the pulse train in the hold phase may be set to be higher than the frequency of the third frequency in the heat phase. In the temperature hold phase, the frequency of the third frequency for selectively heating fat cells is increased to maintain fat cells with a high specific heat at the treatment temperature. Further, pulses adjusted to the second frequency and the first frequency may be applied to the tissue to maintain the epidermal layer and dermal layer at the treatment temperature in the hold phase.

After a predetermined time has elapsed, the controller may stop delivering RF energy. The ratios of frequencies of the pulse trains described above are merely examples, and the controller may configure pulse trains with different frequencies of frequencies in the heat phase and the hold phase.

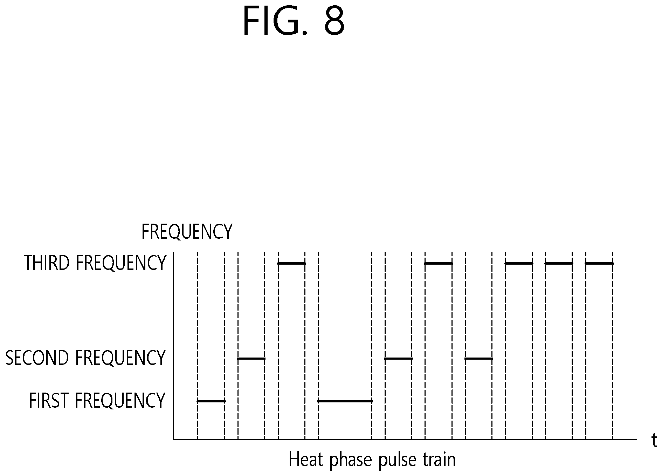

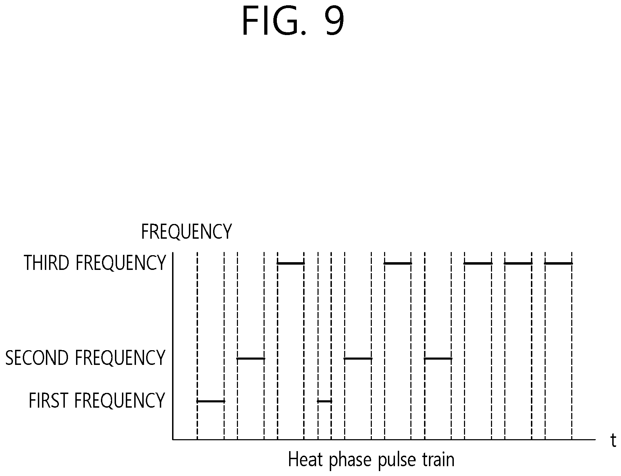

FIGs. 8 and 9 are diagrams showing pulse trains with adjusted heat phase pulse widths in the first embodiment.

The controller may adjust pulse widths on the basis of the temperature of the tissue measured during transmission of RF energy.

Referring to FIG. 8, when delivering RF energy while adjusting the frequency according to the pulse train in the heat phase, the controller can increase the pulse width of the first frequency (2.26 MHz) such that the temperature of the skin surface can increase in a case where a measured temperature of the skin surface is lower than a required temperature.

Referring to FIG. 9, contrary to FIG. 8, in a case where a measured temperature of the skin surface is higher than the required temperature, the controller can prevent excessive temperature increase in the epidermis by reducing the pulse width of the first frequency.

Although FIGs. 6 to 9 show pulse trains in which the frequencies of RF energy pulses are adjusted, the present disclosure can be modified and applied in such a manner that RF energy is adjusted to each frequency and delivered to the tissue as a single pulse.

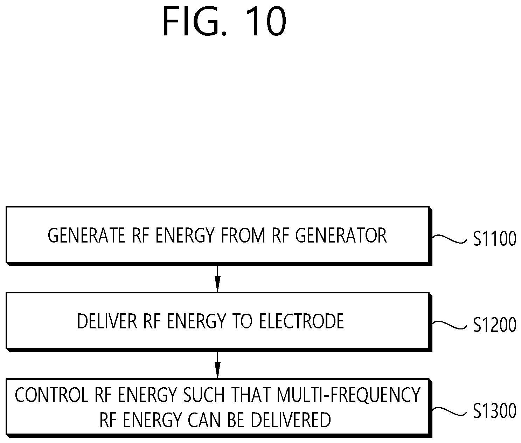

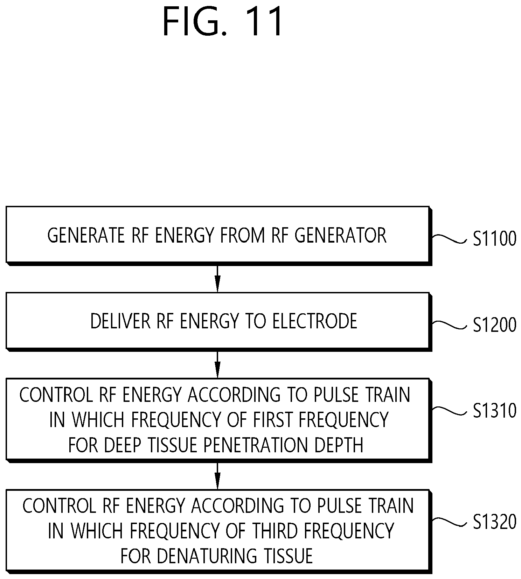

Hereinafter, a method of controlling a skin treatment device for denaturing tissue using multi-frequency RF energy according to a second embodiment of the present disclosure will be described with reference to FIGs. 10 and 11.

FIG. 10 is a flowchart of the method of controlling a skin treatment device for denaturing tissue using multi-frequency RF energy according to a second embodiment of the present disclosure.

Referring to FIG. 10, the method of controlling a skin treatment device for denaturing tissue using multi-frequency RF energy according to the second embodiment of the present disclosure includes a step S1100 of generating RF energy from an RF generator, a step S1200 of delivering the RF energy to an electrode, and a step S1300 of controlling the RF energy such that multi-frequency RF energy can be delivered.

The step S1100 of generating RF energy from the RF generator corresponds to a step of generating RF energy when certain requirements are met according to user input or by a predetermined algorithm. This step can be performed through a controller controlling the RF generator. Here, conditions determined by the controller may be whether the electrode maintains contact with the tissue based on values measured from the electrode.

The step S1200 of delivering the RF energy to the electrode corresponds to a step of delivering the RF energy to the electrode.

The step S1300 of controlling the RF energy such that multi-frequency RF energy can be delivered corresponds to a step of adjusting the frequency and/or pulse width of the RF energy delivered to the electrode. This step may include a step in which the controller controls the RF modulator to adjust the frequency of the RF energy. The controller can adjust the frequency of the RF energy to at least three frequencies for heating different areas in the tissue.

The controller may control the RF energy according to a predetermined pulse train. As an example, first, the controller determines frequencies of a first frequency (2.26 MHz) for heating the epidermis, dermis, and hypodermis, a second frequency (6.75 MHz) for heating the dermis layer, and a third frequency (13.56 MHz) for selectively heating fat and configure a pulse train.

The controller may adjust the frequencies and/or pulse widths of the first to third frequencies constituting the pulse train as treatment time elapses and the temperature of the skin surface increases. The controller may adjust the frequency/pulse width of each frequency in real time. Thereafter, the control unit may perform control such that RF energy is blocked under predetermined conditions, for example, on the basis of the total amount of RF energy delivered or when the treatment time has elapsed.

Although not shown, the controller may adjust the power of the cooling unit to cool the skin surface based on the temperature of the tissue surface and/or the frequency of the applied RF energy.

FIG. 11 is a detailed flowchart of the second embodiment of the present disclosure.

Referring to FIG. 11, in the second embodiment, the step S1300 of controlling the RF energy such that multi-frequency RF energy can be delivered may include a heat phase control step S1310 of controlling the RF energy according to a pulse train in which the frequency of the first frequency for heating a deep portion in the tissue is high, and a hold phase control step S1320 of controlling the RF energy according to a pulse train in which the frequency of the third frequency for denaturing tissue is high.

The heat phase control step S1310 of controlling the RF energy according to a pulse train in which the frequency of the first frequency for heating a deep area in the skin tissue is high is performed to rapidly heat the tissue to a treatment temperature in the initial stage. At this time, if the temperatures of the epidermis, dermis, and hypodermis increase to a certain temperature or higher, the efficiency of RF energy delivery to the deep fat layer increases. Therefore, the controller increases the frequency of the first frequency such that the temperatures of the epidermis layer, dermis layer, hypodermis, and fat layer can rapidly increase to treatment temperatures thereof. Here, the frequency of the first frequency in this step S1310 may be higher than that in the step S1320 which will be described later.

Hold phase control step S1320 involves controlling RF energy according to a pulse train where the frequency of the third frequency for tissue denaturation is high corresponds to a step of controlling the RF energy to maintain the tissue at a treatment temperature. The controller may perform control for maintaining the treatment temperature of fat layer, which has a higher specific heat than the epidermal layer or dermal layer and thus has a slow temperature increase, at the treatment temperature. As an example, the controller may control RF energy by increasing the frequency of selecting 13.56 MHz as the third frequency capable of selectively heating the fat layer. Here, selective heating of the fat layer means that the fat layer is heated because the RF energy absorption rate in fat is higher than that in other layers of the skin tissue when RF energy is delivered at the third frequency.

Consequently, the controller can adjust the frequency of selecting a frequency from among multiple frequencies of RF energy at the beginning and end of RF energy transmission such that optimal treatment can be performed.

The method of controlling a skin treatment device for denaturing tissue using multi-frequency RF energy described above with reference to FIGs. 10 and 11 is applicable to the skin treatment device for denaturing tissue using multi-frequency RF energy according to the first embodiment of the present disclosure described with reference to FIGs. 1 to 9.

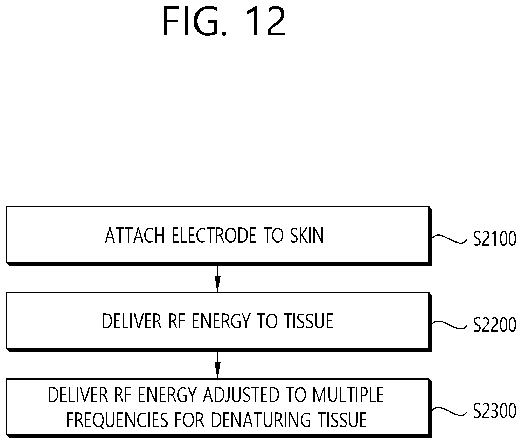

FIG. 12 is a flowchart of a treatment method for denaturing tissue using multi frequency RF energy, which is a third embodiment of the present disclosure.

Referring to FIG. 12, the treatment method for denaturing tissue according to the third embodiment of the present disclosure may include a step S2100 of attaching an electrode to the skin, a step S2200 of delivering RF energy to the tissue, and a step S2300 of adjusting the RF energy adjusted to multiple frequencies for denaturing tissue.

The step S2100 of attaching the electrode to the skin corresponds to a step of attaching the electrode to the tissue determined by a user to require treatment in the patient's skin. The user (or medical staff) selects an area deemed necessary to change the face shape and attaches the electrode capable of delivering RF energy to the skin.

The step S2200 of delivering RF energy to the tissue corresponds to a step of delivering the RF energy to the tissue by user operation in a state in which the electrode is in close contact with the skin.

The step S2300 of delivering the RF energy adjusted to multiple frequencies for denaturing tissue corresponds to a step of controlling heated areas at different depths in the tissue by adjusting the frequency of the RF energy. Skin tissue includes the epidermis, dermis, hypodermis, and fat layer, and the respective layers require different temperatures for treatment. The epidermal layer and dermal layer are degenerated for tissue remodeling, and at this time, the temperature of the tissue for degeneration may be 70 °C to 80 °C. Fat treatment can be performed by heating the fat layer to 44 °C to 46 °C and maintaining the same for a predetermined time to kill the fat cells. This step S2300 is performed to prevent excessive damage to the epidermis and dermis layers and to maintain the fat layer at the treatment temperature. In this step, the multiple frequencies may include the first frequency for heating the epidermis, dermis and hypodermis, the second frequency for heating the dermis layer, and the third frequency for selectively heating fat. In this step, the frequencies of the first frequency, second frequency, and third frequency may vary depending on the treatment mode.

That is, in this step, heating of the epidermis, dermis, and hypodermis is performed at a higher frequency in an early treatment mode, and the frequency of heating the epidermis, dermis, and hypodermis can be lowered in a later treatment mode. Additionally, the frequency of heating the fat layer can be lowered at the beginning of treatment and then increased at the end of treatment.

Further, the third embodiment of the present disclosure may be repeated multiple times to treat the face. That is, the third embodiment of the present disclosure can be performed repeatedly while changing the area to which RF energy is delivered.

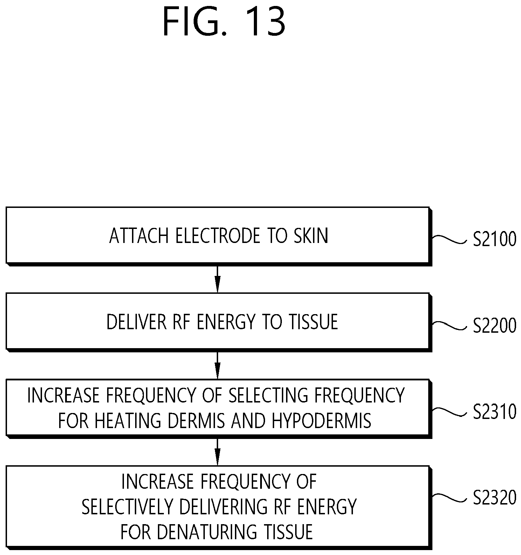

FIG. 13 is a detailed flowchart of the third embodiment.

Referring to FIG. 13, in the third embodiment, the step S2100 of delivering the RF energy to the tissue to treat the fat layer may include a heating step S2310 of increasing the frequency of selecting a frequency for heating a deep area in the tissue, and a holding step S2320 of increasing the frequency of selectively delivering the RF energy to fat cells in the tissue.

The heating step S2310 of increasing the frequency of delivering the RF energy to a deep area in the tissue is a step of rapidly increasing the temperature of the epidermis, dermis, and hypodermis to improve RF energy delivery efficiency according to the pattern in which the RF energy is delivered. At this time, the frequency may be divided into a first frequency region of 1 to 3 MHz, a second frequency region of 3 to 10 MHz, and a third frequency region of 10 to 30 MHz. In this step, the frequency of selecting the frequency of RF energy in the first and second frequency regions may be higher than the frequency of selection in the third frequency region.

The holding step S2320 of increasing the frequency of delivering the RF energy to fat cells in the tissue corresponds to a step of increasing the frequency of the RF energy at the third frequency in order to maintain the temperature of the fat layer at the treatment temperature. At the time of performing this step, RF energy may also be delivered at the first and second frequencies repeatedly to maintain the temperature of the epidermis, dermis, and hypodermis. Meanwhile, the frequency of selecting the third frequency in this step S2320 may be higher than that in the heating step S2310.

The method of denaturing tissue described with reference to FIGs. 12 and 13 may be performed using the skin treatment device for denaturing tissue using multi-frequency RF energy according to the first embodiment of the present disclosure described with reference to FIGs. 1 to 10.

As described above, according to the skin treatment device for denaturing tissue using multi-frequency RF energy, the control method thereof, and the method of denaturing tissue using the same according to the present disclosure, skin treatment can be performed using RF energy having multiple frequencies capable of delivering energy to different depths in the tissue, and thus treatment efficiency can be improved.

## Claims

1. A treatment device for denaturing tissue using RF energy, comprising:
a main body including an RF generator and an RF modulator;
a handpiece connected to the main body and having an electrode provided on one side to deliver RF energy received from the main body to skin; and
a controller configured to control the RF generator and the RF modulator;
**characterized in that** the controller controls the RF modulator to deliver the RF energy according to multiple frequencies.

2. The treatment device of claim 1, wherein the controller controls the RF modulator according to a pulse train including at least three different frequencies of the RF energy.

3. The treatment device of claim 2, wherein the pulse train includes a first frequency, a second frequency, and a third frequency for different heating depths in skin tissue,
wherein the third frequency is selected as a frequency capable of selectively heating fat cells.

4. The treatment device of claim 3, wherein the controller divides an interval in which the RF energy is delivered into a heat phase and a hold phase,
wherein a ratio of the first frequency, the second frequency, and the third frequency constituting the pulse train is set differently in the heat phase and the hold phase.

5. The treatment device of claim 4, wherein the controller configures the pulse train such that a frequency of the first frequency is higher in the heat phase than in the hold phase.

6. The treatment device of claim 4, wherein the controller configures the pulse train such that a frequency of the third frequency is higher in the hold phase than in the heat phase.

7. The treatment device of claim 4, wherein the first frequency is 1 to 3 MHz, the second frequency is 3 to 10 MHz, and the third frequency is 10 to 30 MHz.

8. A method for controlling a treatment device for denaturing tissue using RF energy, the method comprising:
generating RF energy from an RF generator;
delivering the RF energy to an electrode; and
controlling the RF energy,
**characterized in that** the controlling the RF energy according to multiple frequencies.

9. The method of claim 8, wherein the controlling of the RF energy comprises controlling the RF energy according to a pulse train including at least three different frequencies.

10. The method of claim 9, wherein the pulse train includes a first frequency, a second frequency, and a third frequency for different heating depths in skin tissue,
wherein the first frequency is selected as a frequency capable of heating the skin surface to the hypodermis,
the second frequency is selected as a frequency capable of heating the skin surface to the dermis layer, and
the third frequency is selected as a frequency capable of selectively heating fat cells.

11. The method of claim 9, wherein the controlling of the RF energy comprises a heat phase control step and a hold phase control step,
wherein a ratio of the first frequency, the second frequency, and the third frequency constituting the pulse train is set differently in the heat phase control step and the hold phase control step.

12. The method of claim 11, wherein a frequency of the first frequency in the pulse train is higher in the heat phase control step than in the hold phase control step.

13. The method of claim 11, wherein a frequency of the third frequency in the pulse train is higher in the hold phase control step than in the heat phase control step.

14. The method of claim 11, wherein the first frequency is 1 to 3 MHz, the second frequency is 3 to 10 MHz, and the third frequency is 10 to 30 MHz.
